(19) **Europäisches Patentamt**
**European Patent Office**
**Office européen des brevets**

(11) **EP 4 594 276 B1**

(12) **EUROPÄISCHE PATENTSCHRIFT**

(45) Veröffentlichungstag und Bekanntmachung des Hinweises auf die Patenterteilung:
**01.04.2026 Patentblatt 2026/14**

(21) Anmeldenummer: **23776971.6**

(22) Anmeldetag: **26.09.2023**

(51) Internationale Patentklassifikation (IPC):
**C07C 5/48** *(2006.01)*   **C07C 11/04** *(2006.01)*
**C07C 51/215** *(2006.01)*   **C07C 51/25** *(2006.01)*
**C07C 53/08** *(2006.01)*

(52) Gemeinsame Patentklassifikation (CPC):
(C-Sets verfügbar)
**C07C 5/48; C07C 51/215; C07C 51/25;**
C07C 2523/20; C07C 2523/22; C07C 2523/28;
C07C 2527/057                                    (Forts.)

(86) Internationale Anmeldenummer:
**PCT/EP2023/076555**

(87) Internationale Veröffentlichungsnummer:
**WO 2024/068638 (04.04.2024 Gazette 2024/14)**

(54) **VERFAHREN ZUR HERSTELLUNG VON ETHYLEN UND ESSIGSÄURE, VERFAHREN ZUR HERSTELLUNG EINER ZIELVERBINDUNG UNTER VERWENDUNG VON ETHYLEN UND ESSIGSÄURE**

METHOD FOR PRODUCING ETHYLENE AND ACETIC ACID, METHOD FOR PRODUCING TARGET COMPOUND USING ETHYLENE AND ACETIC ACID

PROCÉDÉ DE PRODUCTION D'ÉTHYLÈNE ET D'ACIDE ACÉTIQUE, PROCÉDÉ DE PRODUCTION D'UN COMPOSÉ CIBLE À L'AIDE D'ÉTHYLÈNE ET D'ACIDE ACÉTIQUE

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **26.09.2022 EP 22020458**

(43) Veröffentlichungstag der Anmeldung:
**06.08.2025 Patentblatt 2025/32**

(73) Patentinhaber: **Linde GmbH**
**82049 Pullach (DE)**

(72) Erfinder:
• **MEISWINKEL, Andreas**
**82049 Pullach (DE)**
• **SCHUBERT, Martin**
**82049 Pullach (DE)**
• **TOTA, Desislava**
**82049 Pullach (DE)**
• **ZELLHUBER, Mathieu**
**82049 Pullach (DE)**

(56) Entgegenhaltungen:
**WO-A1-2018/115416     US-A1- 2002 082 445**

(52) Gemeinsame Patentklassifikation (CPC): (Forts.)

C-Sets
**C07C 5/48, C07C 11/04;**
**C07C 51/215, C07C 53/08;**
**C07C 51/25, C07C 53/08**

**Beschreibung**

Die vorliegende Erfindung betrifft ein Verfahren zur Herstellung von Ethylen und Essigsäure und ein Verfahren und eine Anlage zur Herstellung einer Zielverbindung unter Verwendung von Ethylen und Essigsäure gemäß den Oberbegriffen der entsprechenden unabhängigen Patentansprüche.

Hintergrund der Erfindung

Die oxidative Dehydrierung (engl. Oxidative Dehydrogenation, ODH) von Paraffinen mit zwei bis vier Kohlenstoffatomen ist grundsätzlich bekannt. Bei der ODH werden die genannten Paraffine mit Sauerstoff unter anderem zu den jeweiligen Olefinen und zu Wasser umgesetzt. Die vorliegende Erfindung betrifft die oxidative Dehydrierung von Ethan zu Ethylen, nachfolgend auch als ODHE bezeichnet. Ist nachfolgend daher von einer "oxidativen katalytischen Dehydrierung" die Rede, sei hierunter insbesondere eine oxidative katalytische Dehydrierung von Ethan verstanden.

Die ODHE kann gegenüber etablierteren Verfahren zur Herstellung von Olefinen wie dem Steamcracken vorteilhaft sein. So besteht aufgrund der Exothermie der beteiligten Reaktionen und der praktisch irreversiblen Wasserbildung keine thermodynamische Gleichgewichtslimitierung. Die ODHE kann bei vergleichsweise geringen Reaktionstemperaturen durchgeführt werden. Grundsätzlich ist keine Regenerierung der eingesetzten Katalysatoren erforderlich, da die Anwesenheit von Sauerstoff eine in-situ-Regenerierung ermöglicht bzw. bewirkt. Schließlich werden im Gegensatz zum Steamcracken geringere Mengen an wertlosen Nebenprodukten wie Koks gebildet.

Zu weiteren Details bezüglich der ODHE sei auf einschlägige Fachliteratur, beispielsweise Ivars, F. und Löpez Nieto, J. M., Light Alkanes Oxidation: Targets Reached and Current Challenges, in: Duprez, D. und Cavani, F. (Hrsg.), Handbook of Advanced Methods and Processes in Oxidation Catalysis: From Laboratory to Industry, London 2014: Imperial College Press, Seiten 767-834, oder Gärtner, C.A. et al., Oxidative Dehydrogenation of Ethane: Common Principles and Mechanistic Aspects, ChemCatChem, Bd. 5, Nr. 11, 2013, Seiten 3196 bis 3217, sowie X. Li, E. Iglesia, Kinetics and Mechanism of Ethane Oxidation to Acetic Acid on Catalysts Based on Mo-V-Nb Oxides, J. Phys. Chem. C, 2008, 112, 15001-15008, verwiesen.

US 2002/082445 A1 offenbart ein Verfahren zur Oxidation eines C2- bis C4-Alkans zur Herstellung des entsprechenden Alkens und der entsprechenden Carbonsäure, wobei das Verfahren das In-Kontakt-Bringen des Alkans, eines molekularen sauerstoffhaltigen Gases und des entsprechenden Alkens und gegebenenfalls von Wasser in einer Oxidationsreaktionszone in Gegenwart von mindestens einem Katalysator, der für die Oxidation des Alkans zu dem entsprechenden Alken und der Carbonsäure aktiv ist, zur Herstellung eines Produktstroms, der Alken, die Carbonsäure und Wasser aufweist, umfasst. In dem Verfahren wird das Molverhältnis von Alken zu Carbonsäure, das in der Oxidationsreaktionszone erzeugt wird, auf einen vorbestimmten Wert eingestellt oder auf diesem beibehalten, indem die Konzentrationen des Alkens und gegebenenfalls des Wassers in der Oxidationsreaktionszone und gegebenenfalls auch einer oder mehrere der Faktoren Druck, Temperatur und Verweilzeit der Oxidationsreaktionszone gesteuert werden. Ein solches Oxidationsverfahren kann in einem integrierten Prozess verwendet werden, beispielsweise für die Herstellung von Vinylacetat oder Ethylacetat.

Insbesondere haben sich für die ODHE MoVNb-basierte Katalysatorsysteme als vielversprechend herausgestellt, wie beispielsweise bei F. Cavani et al., "Oxidative dehydrogenation of ethane and propane: How far from commercial implementation?", Catal. Today, 2007, 127, 113-131, erwähnt. Auch zusätzlich Te enthaltende Katalysatorsysteme können verwendet werden. Ist hier von einem "MoVNb-basierten Katalysatorsystem" oder einem "MoVTeNb-basierten Katalysatorsystem" die Rede, sei hierunter ein Katalysatorsystem verstanden, das die genannten Elemente als Mischoxid aufweist, auch ausgedrückt mit $MoVNbO_x$ bzw. $MoVTeNbO_x$. Die Angabe von Te in Klammern steht für dessen optionale Anwesenheit. Die vorliegende Erfindung kommt mit solchen Katalysatorsystemen zum Einsatz.

Bei der ODHE wird insbesondere bei Verwendung von $MoVNb(Te)O_x$-basierten Katalysatoren unter industriell relevanten Reaktionsbedingungen eine signifikante Menge an Essigsäure als Nebenprodukt gebildet. Für einen wirtschaftlichen Anlagenbetrieb ist daher eine Koppelproduktion und weitere Verwendung von Ethylen und Essigsäure bei Verwendung des beschriebenen Katalysatortyps in der Regel unvermeidlich und erforderlich. Eine bevorzugte Bildung von Ethylen ist dabei in der technischen Anwendung üblicherweise wünschenswert bzw. die Bildung von Essigsäure ist in herkömmlichen Verfahren unerwünscht. In integrierten petrochemischen Komplexen besteht jedoch oftmals ein Bedarf an genau diesen beiden Wertprodukten. Es existieren wichtige technische Prozesse, die sowohl Ethylen als auch Essigsäure als Edukt benötigen, wie auch unten erläutert. Damit besteht ein Bedarf nach Möglichkeiten zur Herstellung von Ethylen und Essigsäure, insbesondere in einem für entsprechende Prozesse benötigten bedarfsgerechten Verhältnis.

Die vorliegende Erfindung stellt sich vor diesem Hintergrund die Aufgabe, die Herstellung von Ethylen und Essigsäure unter Einsatz einer oxidativen Dehydrierung von Ethan vorteilhaft zu gestalten sowie insbesondere auch die Herstellung von Verfahrensprodukten aus Ethylen und Essigsäure zu verbessern.

Kurzdarstellung der Erfindung

Die vorstehend genannte Aufgabe wird durch Verfahren mit den Merkmalen der jeweiligen unabhängigen Patentansprüche gelöst. Bevorzugte Ausgestaltungen der Erfindung sind jeweils Gegenstand der abhängigen Patentansprüche und der nachfolgenden Beschreibung.

Es wird ein Verfahren zur Herstellung von Ethylen und Essigsäure vorgeschlagen, bei dem Ethan und Sauerstoff in einem Einsatzgemisch unter Erhalt eines Ethylen, Essigsäure und weitere Komponenten enthaltenden Produktgemischs einer oxidativen katalytischen Dehydrierung unter Verwendung eines zumindest Molybdän, Vanadium und Niob sowie optional Tellur als Mischoxid enthaltenden Katalysators unterworfen werden. Zu entsprechenden Katalysatoren, die im Rahmen der vorliegenden Erfindung insbesondere mit entsprechenden Verdünnungsmaterialien verdünnt oder auf geeigneten, insbesondere inerten, Trägern zum Einsatz kommen können, sei auf die obigen Erläuterungen verwiesen. Ein entsprechender Katalysator ist insbesondere schwefelfrei bzw. frei von Schwefelspezies oder weist Schwefel oder Schwefelspezies in einem Gehalt von weniger als 100 Gew.-ppm (Millionstel Gewichtsanteilen), insbesondere weniger als 10 Gew.- ppm, weiter insbesondere weniger als 1 Gew.-ppm, auf. Im Rahmen der vorliegenden Erfindung weist das Einsatzgemisch einen Ethylengehalt von 0,25 bis 30 mol.-% (Molprozent), insbesondere von 0,5 bis 25 mol.-%, weiter insbesondere 1 bis 20 mol.-%, auf, wobei der Gehalt generell, und ungeachtet der jeweiligen Untergrenze, insbesondere bei weniger als 25 mol.-%, weiter insbesondere weniger als 20 mol.-%, noch weiter insbesondere bei weniger als 15 mol.-%, liegen kann.

Das vorgeschlagene Verfahren beruht, wie auch weiter unten erläutert, auf der überraschenden Erkenntnis, dass durch eine Verwendung eines entsprechenden Ethylenanteils in einem Einsatzstrom zur oxidativen katalytischen Dehydrierung der Anteil an Essigsäure im Produktstrom bei Einsatz eines entsprechenden Katalysators signifikant erhöht werden kann. Insbesondere kann hierdurch das Verhältnis von Ethylen zu Essigsäure im Produktstroms gezielt so eingestellt werden, dass dieses dem Bedarf mindestens eines stromabwärtigen Prozesses oder dem mittleren Bedarf mehrerer stromabwärtiger Prozesse entspricht. Diese Einstellung kann dabei weitestgehend dem tatsächlichen Bedarf entsprechen, kleinere Abweichungen bzw. Schwankungen können durch geeignete Zwischenspeicher (Tanks), wie sie üblicherweise ohnehin in einem entsprechenden Komplex vorhanden sind, sowohl für Ethylen als auch für Essigsäure ausgeglichen werden.

In der vorliegenden Erfindung wird zumindest ein Teil des Ethylengehalts des Einsatzgemischs der der oxidativen katalytischen Dehydrierung durch Ethylen gebildet, das in dem Produktstrom der der oxidativen katalytischen Dehydrierung enthalten ist und in die oxidative katalytische Dehydrierung rückgeführt wird. Auf diese Weise ist eine bedarfsgerechte Einstellung des Ethylengehalts ohne eine Verwendung von extern bereitgestelltem Ethylen möglich.

Die Erhöhung bzw. Einstellung des Ethylengehalts im Einsatzgemisch kann über unterschiedliche Anpassungen des Anlagenbetriebs erfolgen, insbesondere in der Form von Betriebsparameteränderungen und/oder Entnahme von Teilströmen im Zerlegungsteil einer entsprechenden Anlage und deren Zumischung in das Einsatzgemisch. Insbesondere kann durch die vorgenommenen Betriebsanpassungen lediglich der Ethylenanteil im Einsatzgemisch nennenswert erhöht werden, während andere Komponentenanteile (insbesondere von Ethan) insbesondere annähernd gleichbleiben bzw. reduziert werden.

In dem vorgeschlagenen Verfahren wird das Produktgemisch oder ein Teil hiervon einer Primäraufbereitung unterworfen, der ein Folgegemisch entnommen wird, das gegenüber dem Produktgemisch an Kohlendioxid und Wasser abgereichert ist.

Das Folgegemisch oder ein Teil hiervon wird als Tieftemperaturtrenneinsatz einer Tieftemperaturtrennung zugeführt, die eine Demethanisierung und insbesondere eine Ethan-Ethylen-Trennung umfasst. Zumindest ein Teil des Tieftemperaturtrenneinsatzes wird als ein Demethanisierungseinsatz der Demethanisierung zugeführt und in der Demethanisierung werden eine schwere Fraktion und eine leichte Fraktion, letztere als Kopffraktion, gebildet. Die schwere Fraktion ist gegenüber dem Folgegemisch an Verbindungen bzw. Kohlenwasserstoffen mit zwei Kohlenstoffatomen (C2-Verbindungen, insbesondere einer Summe aus Ethan und Ethylen) angereichert und an den leichter als Ethylen siedenden Komponenten abgereichert, wohingegen die leichte Fraktion gegenüber dem Folgegemisch an den Verbindungen mit zwei Kohlenstoffatomen abgereichert und an den leichter als Ethylen siedenden Komponenten angereichert ist. Die leichte Fraktion enthält erfindungsgemäß eine signifikante Menge an Ethylen, d.h. 1 bis 90 Mol- oder Volumenprozent.

Das vorgeschlagene Verfahren kann umfassen, dass die schwere Fraktion oder ein Teil hiervon der Ethan-Ethylen-Trennung zugeführt wird. Das Verfahren umfasst aber in jedem Fall, dass die leichte Fraktion oder ein Teil hiervon in die oxidative Dehydrierung rückgeführt wird.

In dem vorgeschlagenen Verfahren wird zusammen mit der üblichen, sogenannten C1-Fraktion, die aus den leichter als Ethylen siedenden Verbindungen besteht, eine gewünschte Menge an Ethylen über den Kopfstrom einer modifizierten Demethanisierung ausgetragen. Als zusätzlicher Vorteil kann hierdurch zusätzlich bereits bei vergleichsweise hohen Temperaturen am Kopf einer derart modifizierten Demethanisierung ein flüssiger, Ethylen enthaltender Rücklauf gewährleistet werden. Zudem ist im Produktstrom der katalytischen oxidativen Dehydrierung der Anteil einer C1-Fraktion bzw. der leichter als Ethylen siedenden Verbindungen üblicherweise vergleichsweise gering. Insbesondere ist hier kein oder nur eine geringe Menge (beispielsweise einem gewissen Anteil, der aus dem Ethaneinsatz der katalytischen oxidativen Dehydrierung stammt) an Methan zu erwarten. Es verbleiben also im wesentlichen Kohlenmonoxid und Ethylen (als dominierende C2-Verbindung) im Kopfstrom dieser modifizierten Demethanisierung. Das Kohlenmonoxid wird dann ebenfalls zur katalytischen oxidativen Dehydrierung rückgeführt und dort zumindest anteilig zu Kohlendioxid umgesetzt. Da sich nun ein deutlich erhöhter Massenstrom am Kopf einer entsprechend modifizierten Demethanisierung ergibt, sind ebenfalls höhere Sauerstoffeintrittskonzentrationen im Einsatzstrom der modifizierten Demethanisierung zulässig, ohne dass eine kritische Anreicherung von Sauerstoff im Kopfstrom erfolgt. In entsprechenden Ausgestaltungen wird also dennoch eine wirksame Vermeidung des Erreichens von explosionsgefährlichen Zusammensetzungen (also einer Sauerstoffkonzentration oberhalb der Sauerstoffgrenzkonzentration, SGK) im Kopfstrom des modifizierten Demethanizers durch Verdünnung mit C2-Verbindungen (insbesondere Ethylen) erzielt.

In einer Ausgestaltung der vorliegenden Erfindung kann ein (insbesondere Mengen- oder Volumen-) Anteil von 1% bis 35% des in dem Produktstrom der der oxidativen katalytischen Dehydrierung insgesamt enthaltenen Ethylens in die oxidative katalytische Dehydrierung rückgeführt werden. Insbesondere können auch Untergrenzen des Anteils von 1%, 2% oder 4% und Obergrenzen von 35%, 30%, 20%, 10% und 5% vorgesehen sein. Derartige Werte haben sich, wie weiter unten belegt, als besonders günstig erwiesen.

Die Entnahme eines Ethylenstromes oder eines Ethan und Ethylen enthaltenden Mischstroms für eine Rückführung könnte grundsätzlich an unterschiedlichen Punkten des Verfahrens ("Entnahmepunkt") erfolgen. Entsprechend - aber nicht als abschließende Aufzählung - sind die nachfolgend erläuterten Ausgestaltungen einsetzbar, wobei das hier vorgeschlagene Verfahren eine Entnahme (auch) am Kopf einer modifizierten Demethanisierung umfasst.

In Ausgestaltungen der vorliegenden Erfindung kann das rückgeführte Ethylen mit in dem Produktgemisch enthaltenem Ethan, insbesondere ohne Trennung voneinander, in die oxidative katalytische Dehydrierung rückgeführt werden. Dies ermöglicht insbesondere die Rückführung entsprechender Mischfraktionen und für diese den Verzicht auf eine komplette Trennung.

Die erwähnten weiteren Komponenten des Produktgemischs in der oxidativen katalytischen Dehydrierung können insbesondere nicht umgesetztes Ethan, leichter als Ethylen siedende Verbindungen, darunter insbesondere Kohlenmonoxid, sowie Kohlendioxid und Wasser umfassen, wobei das, wie erwähnt unter Verwendung einer Primäraufbereitung unter Verwendung des Produktgemischs oder eines Teils hiervon gebildete und gegenüber dem Produktgemisch an Kohlendioxid und Wasser abgereicherte Folgegemisch zumindest einen Teil des Ethans, des Ethylens und der leichter als Ethylen siedenden Verbindungen aus dem Produktgemisch enthält.

Die Primäraufbereitung kann insbesondere eine Kondensatabscheidung unter Bildung einer Wasser-Essigsäure-Fraktion sowie eine Kohlendioxidentfernung und Trocknung umfassen. Ferner sind insbesondere auch eine Verdichtung und Rohgasbehandlung (wie unten erläutert) Teil der Primäraufbereitung. Aus der Wasser-Essigsäure-Fraktion kann insbesondere in einer Essigsäureaufbereitung eine Essigsäurefraktion beliebiger Reinheit gewonnen werden. Entsprechende Aufbereitungsschritte der Primäraufbereitung können in fachüblicher Weise ausgestaltet sein und insbesondere einen "warmen" Teil einer entsprechenden Trennsequenz darstellen.

Eine Ausgestaltung der Erfindung kann insbesondere umfassen, in der Primäraufbereitung eine Rohgasbehandlung, die insbesondere eine katalytische Entfernung von Acetylenen darstellt, sowie eine Kohlendioxidentfernung, insbesondere in Form einer Aminwäsche, und eine, insbesondere adsorptive, Trocknung vorzunehmen, wobei ein Teil eines der Rohgasbehandlung entnommenen Gasgemischs stromauf der Kohlendioxidentfernung und Trocknung aus der Primäraufbereitung ausgeführt und in die oxidative katalytische Dehydrierung rückgeführt werden kann. Kohlendioxid, das in dieser Ausgestaltung noch in einem entsprechenden Gasgemisch enthalten ist, kann in gewissem Umfang als inert in der oxidativen katalytischen Dehydrierung angesehen werden, insbesondere bei dem vorliegend verwendeten Katalysator und den typischen Reaktionstemperaturen. Ein Vorteil ergibt sich hierbei insbesondere in einer Entlastung der Kohlendioxidentfernung, weil dort eine geringere Gasmenge bearbeitet werden muss.

Im Rahmen der vorliegenden Erfindung können für die Tieftemperaturtrennung, die, wie erwähnt, eine Demethanisierung und eine Ethan-Ethylen-Trennung umfasst, unterschiedliche Ausgestaltungen vorgesehen sein, die insbesondere die Reihenfolge dieser Schritte betreffen.

In einer Ausgestaltung kann zumindest ein Teil des Tieftemperaturtrenneinsatzes in einer gegenüber dem in der Primäraufbereitung gebildeten Folgegemisch unveränderten stofflichen Zusammensetzung, aber auch ggf. nach einer Hydrierung oder oder einer anderen katalytischen Umsetzung, insbesondere zur Entfernung von Acetylen, als der Demethanisierungseinsatz der Demethanisierung zugeführt werden. Die Demethanisierung kann in fachüblicher Weise ausgestaltet sein, wird in dem vorgeschlagenen Verfahren jedoch derart modifiziert bzw. betrieben, dass die Kopffraktion bzw. leichte Fraktion (die Begriffe werden hier synonym verwendet), erfindungsgemäß einen gewissen Etylenanteil aufweist.

Dieser Etylenanteil (die nachfolgenden Angaben sind jeweils in Mol- oder Volumenprozent ausgedrückt) beträgt erfindungsgemäß 1 bis 90%, insbesondere 5 bis 75%, beispielsweise 10 bis 50%. Als weitere Komponente ist, in einer nicht notwendigerweise abschließenden Aufzählung, insbesondere Kohlenmonoxid in einem Gehalt von bis zu 75%, insbesondere von bis zu 50%, weiter insbesondere von bis zu 25%, beispielsweise von bis zu 10% enthalten, aber nur wenig oder kein Methan, d.h. ein Methangehalt beträgt insbesondere bis zu 15%, weiter insbesondere bis zu 10%, noch weiter insbesondere bis zu 5%, beispielsweise bis zu 2%. Auch Ethan kann enthalten sein, wobei entsprechende Gehalte bei 0 bis 50%, insbesondere bei 0 bis 30%, weiter insbesondere bei 0 bis 20%, liegen können. Es versteht sich, dass sich die genannten Werte jeweils zu 100% ergänzen können. Sauerstoff ist insbesondere in einem Gehalt unterhalb der SGK enthalten, insbesondere unterhalb von 5, 2,5, 1 oder 0,5%.

Diese Kopffraktion wird in dem vorgeschlagegenen Verfahren daher in die oxidative Dehydrierung zurückgeführt. Auch aus der Sumpffraktion bzw. schweren Fraktion (auch diese Begriffe werden hier synonym verwendet) der Demethanisierung kann zumindest Ethan in die oxidative Dehydrierung zurückgeführt werden. Hierbei kann insbesondere noch ein C2-Splitter bekannter Art zwischengeschaltet sein, also eine zumindest teilweise Trennung von Ethan und Ethylen.

Eine hier erhaltene ethanreiche Fraktion kann dann insbesondere in die oxidative Dehydrierung zurückgeführt werden.

In alternativen Ausgestaltungen ist die Reihenfolge von Demethanisierung und Ethan-Ethylen-Trennung umgekehrt. Hierbei kann zumindest ein Teil des Tieftemperaturtrenneinsatzes, insbesondere in einer gegenüber dem in der Primäraufbereitung gebildeten Folgegemisch unveränderten stofflichen Zusammensetzung, aber auch ggf. nach einer Hydrierung oder einer anderen katalytischen Umsetzung, insbesondere zur Entfernung von Acetylen, als ein Ethan-Ethylen-Trenneinsatz der Ethan-Ethylen-Trennung zugeführt werden, wobei in der Ethan-Ethylen-Trennung eine gegenüber dem in der Primäraufbereitung gebildeten Folgegemisch an Ethan angereicherte und an Ethylen und den leichter als Ethylen siedenden Komponenten abgereicherte schwere Fraktion und eine gegenüber dem in der Primäraufbereitung gebildeten Folgegemisch an Ethan abgereicherte und an Ethylen und den leichter als Ethylen siedenden Komponenten angereicherte leichte Fraktion gebildet werden kann, wobei die leichte Fraktion oder ein Teil hiervon als ein Demethanisierungseinsatz der Demethanisierung zugeführt wird. Eine entsprechende Ausgestaltung ermöglicht insbesondere durch eine angepasste Speisung einer entsprechenden Ethan-Ethylen-Trennung bzw. eine angepasste Entnahme von Komponentengemischen vorteilhafte Effekte.

Somit kann, in einer entsprechenden Ausgestaltung, die Kopffraktion der Demethanisierung einen Teil des in dem in der Primäraufbereitung gebildeten Folgegemisch enthaltenen Ethylens enthalten, und zwar insbesondere in dem bereits erwähnten Umfang, wobei zumindest ein Teil der Kopffraktion zur Bereitstellung zumindest eines Teils des Ethylenanteils des Einsatzgemischs in die oxidative katalytische Dehydrierung rückgeführt werden kann.

Die Entnahme eines Ethan und Ethylen enthaltenden Mischstroms außerhalb der Ethan-Ethylen-Trennung, z.B. eines Teils eines Sumpfstroms der Demethanisierung, und anschließende Zumischung in den typischerweise rückgeführten Ethanstrom vom Sumpf einer Ethan-Ethylen-Trennung ist ebenfalls vorgesehen. Optional können der Mischstrom und/oder der rückgeführte Ethanstrom vor der Vermischung entspannt und beispielsweise gegen abzukühlendes Prozessgas erwärmt werden. Bei einer solchen Betriebsweise müssen bei Veränderung der Ethylenrückführrate vorteilhafterweise keine Veränderungen der Kopf- und Sumpfspezifikationen der Ethan-Ethylen-Trennung vorgenommen werden. Es kommt vor allem zu einer Lastveränderung der Ethan-Ethylen-Trennung (Eintrittsgasmenge) sowie zu einer moderaten Verschiebung der Eintrittsgaszusammensetzung.

In weiteren Ausgestaltungen kann bzw. können auch eine oder mehrere weitere Fraktionen, die aus der in der Ethan-Ethylen-Trennung gebildeten Kopffraktion und der in der Ethan-Ethylen-Trennung gebildeten Sumpffraktion ausgewählt ist oder sind, zumindest zum Teil oder jeweils zumindest zum Teil in die oxidative Dehydrierung rückgeführt werden. Entsprechende Fraktionen, oder rückgeführte Anteile hiervon, können in beliebiger und jeweils geeigneter Weise und in beliebigen Verhältnissen miteinander vereinigt werden.

Die eine oder zumindest eine der mehreren weiteren Fraktionen werden, ebenso wie die aus der in der Demethanisierung gebildete Kopffraktion und die in der Demethanisierung gebildete Sumpffraktion, dabei vorteilhafterweise als oder jeweils als Ethan und Ethylen enthaltende Fraktion oder Fraktionen gebildet. Es handelt sich um Mischfraktionen, die in der zuvor erläuterten Weise erzeugt werden und ggf. auch weitere Komponenten, je nach dem Ort ihrer Bildung, wie beispielsweise Sauerstoff und/oder Kohlenmonoxid enthalten können. Wird ein Teil eines Gasgemischs wie oben erläutert aus der Primäraufbereitung ausgeführt, kann dieses auch insbesondere noch Kohlendioxid enthalten. Aber auch entsprechende Reinfraktionen (Ethan und/oder Ethylenfraktionen) können in entsprechender Weise zurückgeführt werden.

Insbesondere kann auch der Ethan-Ethylen-Trennung ein Ethan und Ethylen enthaltender Seitenstrom entnommen werden, der zumindest zum Teil in die oxidative Dehydrierung rückgeführt wird.

In Ausgestaltungen der Erfindung kann dabei insbesondere auch eine Erhöhung des Ethylenschlupfs am Sumpf der Ethan-Ethylen-Trennung durch teilweise Rücknahme der Aufkocherleistung erfolgen. Dadurch können insbesondere höhere gewünschte Ethylenanteile im Reaktionseinsatz ohne weitere Entnahme- und Zumischungsvorgänge erreicht werden, und es entfallen mögliche weitere Wärmetauscherpassagen für die Anwärmung von ethylenhaltigen Entnahmeströmen.

Besondere Variante ist hier, wenn der Leichtgasschlupf am Sumpf einer Demethanisierung vorgelagerten Ethan-Ethylen-Trennung verwendet wird, da in diesem Fall neben Ethylen auch der Anteil an leichter als Ethylen siedenden Komponenten (insbesondere Kohlenmonoxid und ggf. Methan) im Einsatzgemisch zumindest leicht erhöht wird.

Eine Ausgestaltung der Erfindung kann insbesondere die Entnahme von Ethylen am Kopf der Ethan-Ethylen-Trennung bzw. an beliebiger Stelle in einem optional vorhanden offenen Ethylenkältekreislauf, in den die Ethan-Ethylen-Trennung integriert ist, erfolgen. Die Festlegung der Entnahmestelle erfolgt je nach gewünschtem Aggregatzustand und Druck- und Temperaturniveau des Ethylenstroms. Analog erfolgt die Zumischung zu den sonstigen Reaktionseinsatzströmen möglichst einphasig auf einem ähnlichen Druck- und Temperaturniveau.

Damit kann also ein Teil des in dem Produktgemisch enthaltenen Ethylens in einen offenen Kältemittelkreislauf überführt werden, wobei zumindest ein Teil des Ethylens aus dem offenen Kältekreislauf ausgeführt und zur Bereitstellung zumindest eines Teils des Ethylenanteils des Einsatzgemischs in die in die oxidative katalytische Dehydrierung rückgeführt werden kann.

Die vorstehend erläuterten Rückführungsalternativen können insbesondere im Zusammenhang mit der bereits als Teil der Primäraufbereitung erwähnten Rohgasbehandlung erfolgen, d.h. die erwähnten Primäraufbereitungsschritte können insbesondere eine zumindest teilweise Umsetzung von in dem Produktgemisch enthaltenen Acetylenen und Sauerstoff umfassen. Weitere Ausgestaltungen der Erfindung können aber auch eine Hydrierung stromab einer Demethanisierung und stromauf der Ethan-Ethylen-Trennung sowie stromab der Ethan-Ethylen-Trennung umfassen. In den letzteren Fällen, d.h. bei einer Hydrierung, insbesondere einer sogenannten Tail-End-Hydrierung, wird Sauerstoff im Gegensatz zu einer Rohgasbehandlung typischerweise nicht entfernt, so dass dieser in das rückgeführte Gasgemisch übergehen kann, insbesondere in einer Leichtgasfraktion einer Demethanisierung. In sämtlichen Ausgestaltungen werden vorteilhafterweise nur solche Rückführungen vorgenommen, mittels welcher keine Acetylene rückgeführt werden. Der Fachmann wählt entsprechende Alternativen in geeigneter Weise aus.

In Ausgestaltungen der vorliegenden Erfindung können zumindest ein zur Bereitstellung zumindest eines Teils des Ethylenanteils des Einsatzgemischs in die oxidative katalytische Dehydrierung rückgeführter Stoffstrom oder beliebige andere Stoffströme einer Aufbereitung unterworfen werden, insbesondere einer Abtrennung von Kohlenwasserstoffen mit drei und mehr Kohlenstoffatomen, um auf diese Weise schwerere Komponenten sowohl aus dem Reaktionseinsatz der katalytischen oxidativen Dehydrierung als auch aus dem Recyclestrom abzutrennen. Eine derartige Aufbereitung kann bei Bedarf insbesondere auch gemeinsam mit einem Frischeinsatz durchlaufen werden.

Bei Ethyleneinsatz ist grundsätzliche eine erhöhte Bildung von Kohlenmonoxid und Kohlendioxid in der oxidativen katalytischen Dehydrierung zu erwarten. Hier kann vorteilhaft die ohnehin vorhandene Produktaufreinigung einer entsprechenden Anlage zur oxidativen katalytischen Dehydrierung genutzt werden, die insbesondere eine Demethanisierung und eine Kohlendioxidentfernung umfassen kann.

Ergänzend können im Verfahren weitere Aufreinigungsschritte zum Einsatz kommen, wie eine Spurenentfernung bzw. Rohgasbehandlung und/oder eine Selektivhydrierung von Kohlenwasserstoffen mit zwei Kohlenstoffatomen. Eine Essigsäurefraktion kann durch an sich bekannte Verfahren aufbereitet werden und es kann bedarfsweise Essigsäure entweder in aufkonzentrierter wässriger Lösung oder in Reinform erhalten werden. Damit wird also Essigsäure für entsprechende stromabwärtige Prozesse bereitgestellt. Auch die Ethylenfraktion kann bedarfsgerecht und zumindest anteilig für diese stromabwärtigen Prozesse bereitgestellt werden. Je nach Verfügbarkeit am Standort kann aber auch weiteres Ethylen oder ein ethylenhaltiger bzw. ethylenreicher Strom aus anderen Quellen, wie z.B. einem Steamcracker, verwendet werden.

Die vorliegende Erfindung betrifft auch ein Verfahren zur Herstellung einer Zielverbindung unter Verwendung von Ethylen und Essigsäure, wobei zur Bereitstellung zumindest eines Teils des Ethylens und der Essigsäure ein Verfahren nach einem der vorstehenden Ansprüche verwendet wird. Zu Vorteilen und Ausgestaltungen der Bereitstellung von Ethylen und Essigsäure sei dabei auf die obigen Erläuterungen ausdrücklich verwiesen.

Durch den Einsatz von Ausgestaltungen der Erfindung können für ein nachgeschaltetes Verfahren (ohne Berücksichtigung des Ethylens, welches rückgeführt wird) insbesondere vorteilhafte Verhältnisse von Ethylen zu Essigsäure von weniger als 5:1, weniger als 3:1, weniger als 2:1 oder weniger als 1,5:1 erreicht werden. Insbesondere beträgt das Verhältnis mindestens 0,8:1 bis 1:1.

Die Zielverbindung eines entsprechenden Verfahrens kann insbesondere Vinylacetatmonomer, Ethylacetat, Ethylenvinylacetat oder Polyethylenterephthalat sein. Wie auch unten noch erläutert, besteht in derartigen Verfahren insbesondere ein Bedarf nach Ethylen und Essigsäure in den vorteilhafterweise durch Ausgestaltungen der vorliegenden Erfindung bereitstellbaren Mengenanteilen.

Eine Anlage, die zur Durchführung eines Verfahrens wie zuvor erläutert eingerichtet ist, ist nicht als solche Gegenstand der Erfindung. Auch zu einer entsprechenden Anlage sei auf die obigen Erläuterungen ausdrücklich verwiesen.

Ausgestaltungen der vorliegenden Erfindung werden nachfolgend insbesondere unter Bezugnahme auf die beigefügte Zeichnung erläutert.

Kurze Beschreibung der Zeichnung

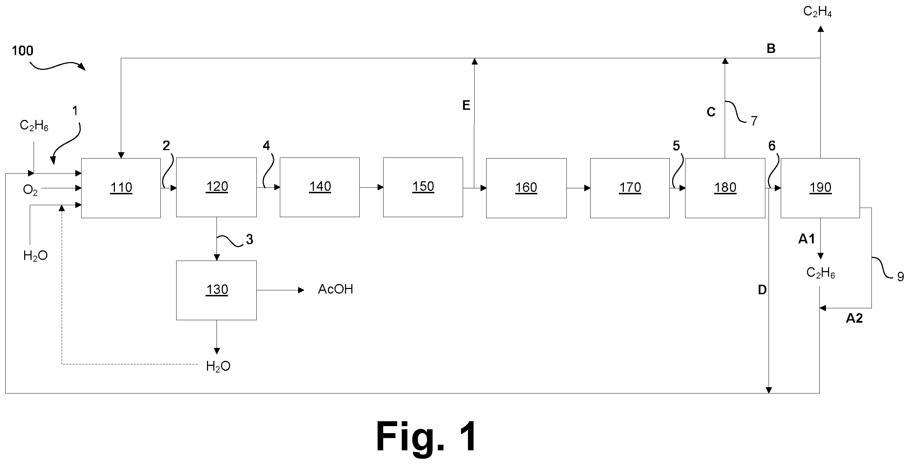

Figuren 1 veranschaulicht ein Verfahren gemäß einer Ausgestaltung der Erfindung in Form eines schematischen Ablaufplans.

Ausführliche Beschreibung

Wird nachfolgend auf Aspekte eines Verfahrens Bezug genommen, betreffen die Erläuterungen entsprechende Anlagen und ihre Ausgestaltungen in gleicher Weise. Entsprechendes gilt für Verfahrensschritte und Anlagenkomponenten. Verfahrensschritte und Anlagenkomponenten gleicher oder vergleichbarer Funktion und/oder technischer Realisierung sind mit identischen Bezugszeichen angegeben.

Das in Figur 1 veranschaulichte Verfahren entspricht einer Ausgestaltung der vorliegenden Erfindung und ist insgesamt mit 100 bezeichnet. In dem Verfahren 100 werden einer katalytischen oxidativen Dehydrierung 110 die benötigten Reaktanden Ethan $C_2H_6$ und Sauerstoff $O_2$ zugeführt, die hier insgesamt mit 1 angegeben sind. Zur Bereitstellung der entsprechenden Stoffströme können auch die weiter unten erläuterten Rückführströme eingesetzt werden. Zusätzlich kann bedarfsweise Wasserdampf $H_2O$ als Verdünnungsmittel in die katalytische oxidative Dehydrierung 110 eingespeist werden. Das dafür benötigte Wasser kann zumindest anteilig aus der Raffinatwasserphase einer weiter unten erläuterten Essigsäureaufreinigung 130 stammen, wie mit einem gestrichelten Pfeil veranschaulicht.

In der katalytischen oxidativen Dehydrierung 110 wird ein Produktgemisch 2 gebildet, das einer Kondensatabscheidung 120 unterworfen wird. In dieser wird eine im Wesentlichen Wasser und Essigsäure enthaltende Kondensatfraktion 3 gebildet. Die Kondensatfraktion 3 wird der erwähnten Essigsäureaufbereitung 130 unterworfen, in der eine im Wesentlichen Wasser enthaltende Wasserfraktion $H_2O$ und eine im Wesentlichen Essigsäure enthaltende Essigsäurefraktion AcOH gebildet werden.

Stromab der Kondensatabscheidung 120 liegt das Produktgemisch 2, nun mit 4 bezeichnet, in an Wasser und Essigsäure abgereicherter Form vor. Es wird nun nacheinander einer Verdichtung 140, einer optionalen Rohgasbehandlung 150, einer Kohlendioxidentfernung 160 und einer Trocknung 170 unterworfen. Die Schritte 120 bis 170 wurden zuvor auch als "Primäraufbereitung" bezeichnet. Stromab dieser liegt ein mit 5 bezeichnetes Komponentengemisch vor, das zuvor auch als Folgegemisch bezeichnet wurde. Dieses enthält zumindest einen Teil des Ethans, des Ethylens und der leichter als Ethylen siedenden Verbindungen aus dem Produktgemisch 4. Es ist gegenüber dem Produktgemisch 4 an Kohlendioxid und Wasser abgereichert bzw. im Wesentlichen frei von diesen Komponenten.

Sodann wird unter Verwendung zumindest eines Teils des in der Primäraufbereitung gebildeten Folgegemischs 5 in einer Demethanisierung 180 ein weiteres Komponentengemisch, das gegenüber dem Folgegemisch 5 an den leichter als Ethylen siedenden Verbindungen abgereichert und an Ethan und Ethylen angereichert ist, gebildet. In der Demethanisierung 180 werden dabei eine Kopffraktion 7 und das genannte weitere Komponentengemisch als Sumpffraktion 6 gebildet, wobei letztere insbesondere einem Kolonnensumpf einer in der Demethanisierung 180 verwendeten Kolonne entnommen wird.

Schließlich werden unter Verwendung zumindest eines Teils der Sumpffraktion 6 in einer Ethan-Ethylen-Trennung 190, die auch als C2-Splitter bezeichnet wird, eine Ethylenfraktion $C_2H_4$, die gegenüber dem der Sumpffraktion 6 an Ethylen angereichert und an Ethan abgereichert ist, und eine Ethanfraktion $C_2H_6$, die gegenüber der Sumpffraktion 6 der Demethanisierung 180 an Ethan angereichert und an Ethylen abgereichert ist, gebildet, wie insoweit für eine Ethan-Ethylen-Trennung üblich.

Mit A1, A2, B, C, D und E sind Rückführströme gemäß Ausgestaltungen der vorliegenden Erfindung veranschaulicht. Jeder Rückführstrom kann, ggf. in Abweichung zur konkreten Darstellung in Figur 1, einzeln oder aber auch beliebiger Kombination mit anderen Strömen zurückgeführt werden.

Mit A1 ist dabei die Ethanfraktion aus der Ethan-Ethylen-Trennung bezeichnet, die zur weiteren Umsetzung des Ausgangsstoffs Ethan in die oxidative katalytische Dehydrierung 110 zurückgeführt wird. Diese kann, wie mit A2 bezeichnet, mit einem über einen Zwischenabzug der Ethan-Ethylen-Trennung 190, beispielsweise einer in der Ethan-Ethylen-Trennung 190 verwendeten Rektifikationskolonne, entnommenen Stoffstrom 9, der einen gewissen und insbesondere über die Entnahmestelle einstellbaren Ethylengehalt aufweist, vereinigt und zusammen mit diesem in die oxidative katalytische Dehydrierung 110 zurückgeführt werden kann. Generell ist immer dann, wenn eine Vereinigung unterschiedlicher Stoffströme vor einer Rückführung in die oxidative katalytische Dehydrierung 110 beschrieben wird, alternativ auch eine separate Rückführung möglich.

Stoffstrom B stellt einen Teil der Ethylenfraktion aus der Ethan-Ethylen-Trennung 190 dar, der insbesondere alternativ oder zusätzlich zu der mit A2 bezeichneten Ausgestaltung in die oxidative katalytische Dehydrierung 110 rückgeführt werden kann. Er kann dabei mit der Kopffraktion 7 aus der Demethanisierung 180 vereinigt werden, wie mit C angegeben. Die Demethanisierung 180 kann dabei insbesondere, abweichend von einer üblichen Demethanisierung, derart betrieben werden, dass neben leichter als Ethylen siedenden Komponenten auch ein Teil des Ethylens in die Kopffraktion 7 übergeht. Wie mit D bezeichnet, kann aber auch ein Teil der Sumpffraktion 9 aus der Demethanisierung 8, die Ethan und Ethylen enthält, rückgeführt werden. Wie auch in anderen Fällen ist eine beliebige Vereinigung entsprechender Stoffströme vor der Rückführung möglich.

Stoffstrom E stellt einen Teil eines der Rohgasbehandlung 150 entnommenen Gasgemischs dar, das stromauf der Kohlendioxidentfernung 160 und Trocknung 170 aus der Primäraufbereitung 120-170 ausgeführt und in die oxidative katalytische Dehydrierung 110 rückgeführt wird.

Vor der Erläuterung von spezifischen Aspekten der vorliegenden Erfindung und ihrer Ausgestaltungen seien nachfolgend zur Einordnung nochmals Grundlagen der Erfindung und weitere Aspekte erläutert.

Wie erwähnt, entsteht insbesondere beim Einsatz von $MoVNbTeO_x$-Katalysatoren in der oxidativen katalytischen Dehydrierung unter industriell relevanten Reaktionsbedingungen neben Ethylen Essigsäure als Koppelprodukt. Dabei werden unter großtechnisch relevanten Bedingungen typischerweise Verhältnisse von Ethylen zu Essigsäure von ca. 5 bis 10 mol/mol erzielt. Der Bedarf an Essigsäure ist jedoch oftmals limitiert auf spezielle stromabwärtige Produkte (z.B. Vinylacetat-Monomer). Essigsäure kann als Bestandteil der wässrigen Kondensatphase (typischer Essigsäuregehalt im Kondensat: 1 bis 30 Gew.-%, 3 bis 25 Gew.-% oder 5 bis 20 Gew.-%) abgetrennt werden und grundsätzlich durch eine geeignete Aufbereitung als eigenes Wertprodukt bereitgestellt werden.

Eine Einstellung des Verhältnisses von Ethylen zu Essigsäure im Produktstrom der oxidativen katalytischen Dehydrierung unter Verwendung von $MoVNbTeO_x$-Katalysatoren ist insbesondere durch eine Anpassung des Wasserpartialdrucks im Edukt, aber insbesondere im Reaktoraustrittsstrom, der Raumgeschwindigkeit sowie des Betriebsdrucks möglich, jedoch nur innerhalb gewisser Grenzen (siehe z.B. WO 2018/115416 A1, EP 3 519 377 B1 sowie WO 2019/243480 A1). Die vorliegende Erfindung ermöglicht hingegen die freie Einstellung anderer Verhältnisse im erläuterten Umfang. Ein Mindestwasseranteil ist zur Gewährleistung einer stabilen Katalysatorperformance vorteilhaft (siehe z.B. WO 2018/115418 A1, EP 3 558 910 B1 sowie WO 2019/243480 A1. Mit Hilfe eines speziell angepassten Reaktordesigns in Verbindung mit optimierten Betriebsbedingungen (z.B. Raumgeschwindigkeit) und/oder Lineargeschwindigkeit kann zwar eine Erhöhung der Ethylenausbeute (siehe US 10,017,432 B2 und US 9,963,412 B2) erreicht werden. Jedoch ist selbst hier der Essigsäureanteil im Produkt noch zu hoch, um diese zu verwerfen. Somit ist eine Verwendung von Essigsäure als Produkt unabdingbar und es gilt bedarfsgerechte Essigsäuremengen bereitzustellen.

Der Einsatz der oxidativen katalytischen Dehydrierung ist aufgrund der Koppelproduktion zumeist begrenzt auf kleine bis mittlere Anlagenkapazitäten und erfordert eine entsprechende Verwertung der produzierten Essigsäure. Andererseits entstehen im Gegensatz zum Steamcracker bei der oxidativen katalytischen Dehydrierung (im Wesentlichen) nur Ethylen und Essigsäure als Produkte. Somit entfällt auch die Anforderung einer geeigneten Verwertung weiterer Produktfraktionen und der entsprechenden apparativen Einrichtungen im Zerlegungsteil. Die vorliegende Erfindung nutzt nun die Bildung von Essigsäure bei der oxidativen katalytischen Dehydrierung bewusst aus und stellt beide Koppelprodukte vorteilhaft bereit.

Eine Anlage zur oxidativen katalytischen Dehydrierung umfasst typischerweise die bereits zu Figur 1 beschriebenen wesentlichen Prozessschritte, die in geeigneter Weise angeordnet sein können. Neben der oxidativen katalytischen Dehydrierung kann eine Kondensatabscheidung 120, eine Essigsäureaufbereitung 130, eine Verdichtung 140 und eine Kohlendioxidentfernung 160 (insbesondere Aminwäsche und ggf. Laugewäsche zur Feinreinigung) sowie eine Ethan-Ethylen-Trennung 190. Des Weiteren können bedarfsweise weitere Prozessschritte wie eine Demethanisierung 180, sowie eine Spurenentfernung bzw. Rohgasbehandlung (auch unter Einspeisung von Wasserstoff und Sauerstoff, siehe z.B. WO 2020/187572 A1) sowie eine Selektivhydrierung von Kohlenwasserstoffen mit zwei Kohlenstoffatomen an geeigneter Stelle eingesetzt werden. Auch eine Trennung von Kohlenwasserstoffen mit zwei und drei Kohlenstoffatomen kann eingesetzt werden, wobei diese typischerweise stromauf der katalytischen oxidativen Dehydrierung angeordnet sein kann, um schwerere Komponenten aus dem Einsatzgemisch der ODHE abzutrennen.

Von Bedeutung im Rahmen der vorliegenden Erfindung ist insbesondere die Essigsäureaufbereitung. Wie eingangs erwähnt, ist die Essigsäure Bestandteil der Kondensatphase. Gemäß Stand der Technik wird die Essigsäure vorteilhafter Weise extraktiv aus der Kondensatphase gewonnen. Als Extraktionsmittel eignen sich organische Lösungsmittel insbesondere aus der Stoffgruppe der Ether, wie z.B. Methyl-tert-Butylether, oder der Stoffgruppe der Ester, wie z.B. Essigsäureethylester. Aus der so gewonnenen organischen Extraktphase wird dann die Essigsäure destillativ abgetrennt, wobei das Extraktionsmittel für weitere Extraktionszyklen zurückgewonnen wird. Bei Bedarf kann die so gewonnene Essigsäure noch einmal bis zur gewünschten Reinheit (z.B. Eisessig) einer Feindestillation unterworfen werden. Die ebenfalls beim Extraktionsschritt anfallende wässrige Raffinatphase kann ebenfalls einer destillativen Aufarbeitung unterzogen werden, um in der Raffinatphase enthaltenes Extraktionsmittel zurückzugewinnen bzw. auch um ein möglichst wenig belastetes Abwasser zu erhalten. Durch eine Essigsäureaufbereitung kann Essigsäure insbesondere in einer Reinheit von mehr als 95%, mehr als 98% oder mehr als 99% und/oder in Eisessigqualität, d.h. in einer Reinheit von mindestens 99,8%, gewonnen und für Folgeprozesse genutzt werden.

Die Herstellung von Essigsäure durch andere Prozesse ist grundsätzlich wohlbekannt und technisch etabliert (C. Le Berre, P. Serp, P. Kalck, G. P. Torrence, "Acetic Acid" in Ullmann's Encyclopedia of Industrial Chemistry 2014). Dabei kommt traditionellerweise und als besonders wichtige Route die Umsetzung von Methanol mit Kohlenmonoxid zum Einsatz. Auch Flüssigphasenoxidationen von z.B. Butan, Naphtha oder Acetaldehyd sind bekannt. Der zitierte Artikel erwähnt ferner Ansätze zur katalytischen Oxidation von Ethylen, dabei wird insbesondere der Einfluss eines Pd-Zusatzes zum Katalysator hervorgehoben.

Auch oxidative Verfahren ausgehend von Ethan über geeignete Mischmetalloxid-Katalysatoren (auf Basis von Mo, V und/oder Nb) wurden bereits entwickelt und kommerzialisiert. Dabei kommen spezielle Modifier zum Einsatz. Beispielsweise schlägt die US 5,907,056 A P, B, Hf, Te und As als Modifier vor und geht ausschließlich von Ethan als Einsatz zu einer oxidativen katalytischen Dehydrierung aus. Die Beispiele in dieser Schrift erreichen Ethanumsätze bis zu ca. 66% bei Verhältnissen von Ethylen zu Essigsäure zwischen ca. 1:1 und 5:1. Lediglich für die Einträge 7 und 8 in Tabelle 3 der Schrift werden Verhältnisse von Ethylen zu Essigsäure von ca. 1,9:1 bzw. 5:1 erreicht, dabei wird jedoch ein P-modifizierter Katalysator verwendet. Die US 6,258,992 A offenbart zwar Feedströme, die neben Alkanen auch Alkene wie Ethylen enthalten können, jedoch enthält der dort offenbarte Katalysator zwingend immer auch eine Schwefelspezies. Die Beispiele in der Schrift sind auf Umsätze von weniger als 20% begrenzt (ausgehend von reinem Ethan), wobei die resultierenden Verhältnisse von Ethylen zu Essigsäure zwischen 1:1 und 1:7 betragen. Gemäß weiteren Anmeldungen, wie z.B. gemäß der EP 1,140,355 B1, werden weitere Promotoren zugesetzt, insbesondere das (teure) Edelmetall Pd, um eine Selektivitätssteigerung in Bezug auf Essigsäure zu bewirken. Die Beispiele in dieser Schrift beschränken sich jedoch auf Paraffine als Einsatzstrom der oxidativen katalytischen Dehydrierung, dabei wird neben Ethan auch Propan untersucht. Auch die US 6,030,920 A umfasst den Zusatz von Pd zu einem MoVNbO$_x$-Katalysator und beschränkt sich explizit auf die Umsetzung von Ethan als Einsatzstrom einer entsprechenden Selektivoxidation.

Prozesse, die sowohl Ethylen als auch Essigsäure als Edukt benötigen, haben dabei einen Bedarf an beiden Edukten in einem definierten Verhältnis. Dieses Verhältnis von Ethylen zu Essigsäure (auf molarer Basis) beträgt z.B. insbesondere für die Produktion von Vinylacetatmonomer 3:1 bis 1:1, von Ethylacetat 3:1 bis 1:1 und von Ethylenvinyl-lacetat von 12:1 bis 8:1. Ethylenvinylacetat kann ausgehend von Vinylacetatmonomer durch Umsetzung mit Ethylen gewonnen werden, wodurch sich das recht hohe Verhältnis von Ethylen zu Essigsäure erklärt, bzw. ist das Verhältnis zusätzlich durch den Grad an Copolymerisation zwischen Ethylen und Vinylacetatmonomer bestimmt.

Ein weiterer Prozess ist die Herstellung von Polyethylenterephthalat via Therephthalsäure, wobei ein Verhältnis von Ethylen zu Essigsäure von 14:1 bis 11:1 erforderlich ist und Essigsäure als Lösungsmittel benötigt wird. Prinzipiell wird die Essigsäure dabei zwar rückgeführt, aufgrund der harschen Reaktionsbedingungen bei der katalytischen Oxidation von p-Xylol (typischerweise mit Luftsauerstoff bei 175 bis 225 °C und 15 bis 30 bara) kommt es zu einem nicht unerheblichen Verlust des Lösungsmittels. Die Verluste betragen bis zu 0,05 Tonnen Essigsäure pro Tonnen Polyethylenterephthalat.

Die klassische katalytische oxidative Dehydrierung von Ethan ausgehend von Ethan kann jedoch, wie erwähnt, Ethylen und Essigsäure nur in einem bestimmten Verhältnis bereitstellen, welches nur bedingt durch Wahl der Prozessbedingungen, insbesondere des Wasseranteiles im Reaktionseinsatz, der Raumgeschwindigkeit sowie des Betriebsdrucks und ggf. eines speziellen Reaktordesigns beeinflussbar ist. Insbesondere ein hoher Essigsäure-Anteil - wie insbesondere für die Downstreamprodukte Vinylacetatmonomer oder Ethylacetat benötigt - kann somit nicht erreicht werden. Es muss also mehr Ethylen erzeugt werden als eigentlich benötigt, was nicht immer erwünscht ist, insbesondere, wenn kein weiterer Ethylenbedarf an einem entsprechenden Standort oder in einem entsprechenden Verbund besteht. Alternativ kann nach Stand der Technik Essigsäure zugekauft oder mittels anderer Verfahren erzeugt werden, was jedoch zusätzlichen Aufwand bedeutet.

Zwar ist eine Erhöhung des Essigsäureanteils in Richtung eines Verhältnisses von Ethylen zu Essigsäure von deutlich kleiner als 5:1 (bevorzugt 3:1 oder kleiner) grundsätzlich denkbar. Dies geht allerdings nicht ohne deutliche Erhöhung des Druckdesigns des Reaktors sowie ergänzend einer Verkleinerung der Anlagenkapazität einher, um den gesteigerten Sicherheitsanforderungen, die sich aus einem erhöhten Sauerstoffbedarf sowie des deutlich erhöhten Drucks ergeben. Das heißt, ein deutlich kleineres molares Produktverhältnis von Ethylen zu Essigsäure als 5:1 kann nicht mit einem Reaktordesign erreicht werden, mit dem ein Bereich von Ethylen zu Essigsäure von ca. 5:1 bis 10:1 hingegen leicht eingestellt werden kann.

Insgesamt führt dies zu einer deutlich höheren Komplexität und somit deutlich höheren Kosten, was die Wirtschaftlichkeit in Frage stellen würde.

Die vorliegende Erfindung offenbart also eine Lösung, um das Verhältnis von Ethylen zu Essigsäure bedarfsgerecht und flexibel mit einem einzigen technisch etablierten Reaktordesign einstellen zu können, insbesondere für einen erhöhten Bedarf an Essigsäure. Ausgestaltungen der vorliegenden Erfindung sind neben bereits bekannten Maßnahmen wie z.B. einer Änderung des Wasseranteils im Reaktionseinsatz, der Raumgeschwindigkeit, der Lineargeschwindigkeit oder des Betriebsdrucks der oxidativen katalytischen Dehydrierung einsetzbar und offenbaren zusätzlich zu derartigen Maßnahmen eine einfache Anpassung an ein anderes benötigtes Verhältnis von Ethylen zu Essigsäure, insbesondere durch Anpassung der Ethylenrückführung bei sonst gleichen apparativen Anforderungen und Betriebsbedingungen.

Der prozentuale Anteil des rückgeführten Ethylens ("R_C2H4"), nachfolgend auch als PRE-Wert bezeichnet, an der Gesamtmenge an rückgeführten Kohlenwasserstoffen mit zwei Kohlenstoffatomen ("R_G"), d.h. der Summe an Ethan und Ethylen, jeweils in mol bzw. kmol, lässt sich wie folgt angeben:

$$PRE = n(R\_C2H4) / n(R\_G) \times 100\%$$

Unter den theoretischen Annahmen, dass sämtliches in der oxidativen katalytischen Dehydrierung nicht umgesetzte Ethan im Produktgemisch ("P_C2H6") wieder zur oxidativen katalytischen Dehydrierung zurückgefahren wird (technisch bedingte Verluste z.B. in Purgeströmen oder Restgehalte in anderen Prozessströmen werden also vernachlässigt) und der Recycle keine weiteren Komponenten wie z.B. geringe Anteile von höheren Kohlenwasserstoffen und/oder Kohlenmonoxid enthält, gilt, wobei die Angabe "R_C2H6" für das rückgeführte Ethan steht:

$$n(R\_G) = n(R\_C2H6) + n(R\_C2H4) = n(P\_C2H6) + n(R\_C2H4)$$

Geht man von einem typischen Ethanumsatz in der oxidativen katalytischen Dehydrierung von 50% aus (technisch übliche Werte liegen im Bereich von ca. 40 bis 60%) ergeben sich also die in Tabelle 1 angegebenen Werte. Dabei berücksichtigt der Wert n(P_C2H4) nur das in der oxidativen katalytischen Dehydrierung aus Ethan gebildete Ethylen und beinhaltet nicht das nicht umgesetzte Ethylen. Dieser Wert dient also nur zur Orientierung und ist real nicht separat messbar. n(R_C2H4) ist erfindungsgemäß aber stets kleiner als n(P_C2H4). Der Wert n(F_C2HG6) gibt den Ethangehalt des Einsatzgemischs an.

**Tabelle 1**

| n(F_C2H6) | Umsatz Ethan | Selektivität Ethan zu Ethylen | n(R_C2H6) | n(P_C2H4) | n(R_C2H4) | PRE |
|---|---|---|---|---|---|---|
| 100 kmol | 40% | 80% | 60,0 kmol | 32,0 kmol | 1,0 kmol | |
| 100 kmol | 40% | 85% | 60,0 kmol | 34,0 kmol | 1,0 kmol | 1,64% |
| 100 kmol | 40% | 90% | 60,0 kmol | 36,0 kmol | 1,0 kmol | |
| 100 kmol | 40% | 80% | 60,0 kmol | 32,0 kmol | 2,5 kmol | |
| 100 kmol | 40% | 85% | 60,0 kmol | 34,0 kmol | 2,5 kmol | 4,00% |
| 100 kmol | 40% | 90% | 60,0 kmol | 36,0 kmol | 2,5 kmol | |
| 100 kmol | 40% | 80% | 60,0 kmol | 32,0 kmol | 5,0 kmol | |
| 100 kmol | 40% | 85% | 60,0 kmol | 34,0 kmol | 5,0 kmol | 7,69% |
| 100 kmol | 40% | 90% | 60,0 kmol | 36,0 kmol | 5,0 kmol | |
| 100 kmol | 40% | 80% | 60,0 kmol | 32,0 kmol | 10,0 kmol | |
| 100 kmol | 40% | 85% | 60,0 kmol | 34,0 kmol | 10,0 kmol | 14,29% |
| 100 kmol | 40% | 90% | 60,0 kmol | 36,0 kmol | 10,0 kmol | |
| 100 kmol | 40% | 80% | 60,0 kmol | 32,0 kmol | 15,0 kmol | |
| 100 kmol | 40% | 85% | 60,0 kmol | 34,0 kmol | 15,0 kmol | 20,00% |
| 100 kmol | 40% | 90% | 60,0 kmol | 36,0 kmol | 15,0 kmol | |
| 100 kmol | 40% | 80% | 60,0 kmol | 32,0 kmol | 20,0 kmol | |
| 100 kmol | 40% | 85% | 60,0 kmol | 34,0 kmol | 20,0 kmol | 25,00% |
| 100 kmol | 40% | 90% | 60,0 kmol | 36,0 kmol | 20,0 kmol | |
| 100 kmol | 50% | 80% | 50,0 kmol | 40,0 kmol | 1,0 kmol | |
| 100 kmol | 50% | 85% | 50,0kmol | 42,5 kmol | 1,0 kmol | 1,96% |
| 100 kmol | 50% | 90% | 50,0 kmol | 45,0 kmol | 1,0 kmol | |
| 100 kmol | 50% | 80% | 50,0 kmol | 40,0 kmol | 2,5 kmol | |
| 100 kmol | 50% | 85% | 50,0 kmol | 42,5 kmol | 2,5 kmol | 4,76% |
| 100 kmol | 50% | 90% | 50,0 kmol | 45,0 kmol | 2,5 kmol | |

(fortgesetzt)

| n(F_C2H6) | Umsatz Ethan | Selektivität Ethan zu Ethylen | n(R_C2H6) | n(P_C2H4) | n(R_C2H4) | PRE |
|---|---|---|---|---|---|---|
| 100 kmol | 50% | 80% | 50,0 kmol | 40,0 kmol | 5,0 kmol | |
| 100 kmol | 50% | 85% | 50,0 kmol | 42,5 kmol | 5,0 kmol | 9,09% |
| 100 kmol | 50% | 90% | 50,0 kmol | 45,0 kmol | 5,0 kmol | |
| 100 kmol | 50% | 80% | 50,0 kmol | 40,0 kmol | 10,0 kmol | |
| 100 kmol | 50% | 85% | 50,0 kmol | 42,5 kmol | 10,0 kmol | 16,67% |
| 100 kmol | 50% | 90% | 50,0 kmol | 45,0 kmol | 10,0 kmol | |
| 100 kmol | 50% | 80% | 50,0 kmol | 40,0 kmol | 15,0 kmol | |
| 100 kmol | 50% | 85% | 50,0 kmol | 42,5 kmol | 15,0 kmol | 23,08% |
| 100 kmol | 50% | 90% | 50,0 kmol | 45,0 kmol | 15,0 kmol | |
| 100 kmol | 50% | 80% | 50,0 kmol | 40,0 kmol | 20,0 kmol | |
| 100 kmol | 50% | 85% | 50,0 kmol | 42,5 kmol | 20,0 kmol | 28,57% |
| 100 kmol | 50% | 90% | 50,0 kmol | 45,0 kmol | 20,0 kmol | |
| 100 kmol | 60% | 80% | 40,0 kmol | 48,0 kmol | 1,0 kmol | |
| 100 kmol | 60% | 85% | 40,0 kmol | 51,0 kmol | 1,0 kmol | 2,44% |
| 100 kmol | 60% | 90% | 40,0 kmol | 54,0 kmol | 1,0 kmol | |
| 100 kmol | 60% | 80% | 40,0 kmol | 48,0 kmol | 2,5 kmol | |
| 100 kmol | 60% | 85% | 40,0 kmol | 51,0 kmol | 2,5 kmol | 5,88% |
| 100 kmol | 60% | 90% | 40,0 kmol | 54,0 kmol | 2,5 kmol | |
| 100 kmol | 60% | 80% | 40,0 kmol | 48,0 kmol | 5,0 kmol | |
| 100 kmol | 60% | 85% | 40,0 kmol | 51,0 kmol | 5,0 kmol | 11,11% |
| 100 kmol | 60% | 90% | 40,0 kmol | 54,0 kmol | 5,0 kmol | |
| 100 kmol | 60% | 80% | 40,0 kmol | 48,0 kmol | 10,0 kmol | |
| 100 kmol | 60% | 85% | 40,0 kmol | 51,0 kmol | 10,0 kmol | 20,00% |
| 100 kmol | 60% | 90% | 40,0 kmol | 54,0 kmol | 10,0 kmol | |
| 100 kmol | 60% | 80% | 40,0 kmol | 48,0 kmol | 15,0 kmol | |
| 100 kmol | 60% | 85% | 40,0 kmol | 51,0 kmol | 15,0 kmol | 27,27% |
| 100 kmol | 60% | 90% | 40,0 kmol | 54,0 kmol | 15,0 kmol | |
| 100 kmol | 60% | 80% | 40,0 kmol | 48,0 kmol | 20,0 kmol | |
| 100 kmol | 60% | 85% | 40,0 kmol | 51,0 kmol | 20,0 kmol | 33,33% |
| 100 kmol | 60% | 90% | 40,0 kmol | 54,0 kmol | 20,0 kmol | |

Aufgrund der Anwesenheit geringer Mengen anderer Komponenten wie höherer Kohlenwasserstoffe und/oder Kohlenmonoxid, liegt der tatsächliche PRE-Wert in der Realität etwas niedriger. Da auch Ethylen als Wertprodukt genutzt und insbesondere bedarfsgerechte Verhältnisse von Ethylen zu Essigsäure erzielt werden sollen, liegen besonders vorteilhafte PRE-Werte also in dem bereits zuvor erwähnten Bereich. Hierdurch können für ein der oxidativen katalytischen Dehydrierung nachgeschaltetes -Verfahren (also ohne Berücksichtigung des Ethylens, welches in den Recycle geführt wird) insbesondere die erwähnten vorteilhaften Verhältnisse von Ethylen zu Essigsäure erreicht werden.

In einer Versuchsanlage wurden Umsetzungen von Ethan sowie von Ethan-Ethylen-Einsätzen unter Bedingungen der oxidativen katalytischen Dehydrierung untersucht. Der entsprechende Versuchsreaktor ist als Doppelrohr ausgeführt und weist eine nutzbare Länge von 0,9 m sowie einen Innendurchmesser des Reaktionsraumes von 10 mm auf. Die Beheizung bzw. Kühlung erfolgt mit Hilfe eines Thermoölbades, wobei das Thermoöl durch den Außenraum des Reaktors gepumpt wird und somit den Innenraum/Reaktionszone beheizt bzw. auch gleichzeitig kühlt (die Umsetzung ist eine exotherme Reaktion). Aufgrund des sehr guten Wärmeübergangs entspricht die Ölbadtemperatur der Eintrittstemperatur des Gases auf das Katalysatorbett (und wurde zudem auch messtechnisch nachgewiesen) und ist somit die Reaktionstemperatur. Die Versuchsbedingungen und die damit erzielten Ergebnisse sind in Tabelle 2 wiedergegeben.

Es ist zu erkennen, dass sich die Selektivität deutlich bei Vorhandensein von Ethylen im Reaktionseinsatz hin zu Essigsäure verschiebt.

**Tabelle 2:** Versuchsbedingungen und Ergebnisse der oxidativen Umsetzung von Ethan und einer Ethan-Ethylen-Mischung.

| | Einheit | Versuch 1 | Versuch 2 |
|---|---|---|---|
| **Versuchsbedingungen** | | | |
| **Kohlenwasserstoffbelastung** | $(mmol/h)_{KW}/kg_{Kat}$ | 26,7 | 23,0 |
| **Anteil Ethylen am Kohlenwasserstoffeinsatz** | % | 0 | 47,7 |
| **Verhältnis Wasser : Kohlenwasserstoffeinsatz** | mol/mol | 1,18 | 0,39 |
| **Betriebsdruck** | bara | 4,9 | 3,1 |
| **Reaktionstemperatur** | °C | 334 | 317 |
| **Ergebnisse** | | | |
| **Ethanumsatz** | % | 55 | 42 |
| **Ethylenselektivität** | % | 74,5 | 51,9 |
| **Essigsäureselektivität** | % | 20,9 | 36,5 |
| **Selektivität zu Kohlenstoffoxiden** | % | 4,6 | 11,4 |
| **Produktverhältnis Ethylen : Essigsäure** | mol/mol | 3,6 | 1,4 |

Die vorliegende Erfindung ermöglicht in ihren Ausgestaltungen insbesondere eine bedarfsgerechte Optimierung und Anpassung der Ethylen- und Essigsäure-Kapazität über die üblichen Limitierungen bei reinem Ethaneinsatz hinaus. Eine flexible Anpassung des Verhältnisses von Ethylen zu Essigsäure ist insbesondere hin zu hohen Essigsäure-Anteilen möglich. Insbesondere ist dies auch über ein Verhältnis hinaus möglich, dass allein durch Variation der Prozessparameter der oxidativen katalytischen Dehydrierung alleine erreicht werden kann.

Hierdurch ermöglicht die vorliegende Erfindung einen vorteilhaften Einsatz der katalytischen oxidativen Dehydrierung auch für höhere Essigsäure-Kapazitäten unabhängig vom spezifischen Ethylenbedarf. Damit entfallen bisher verbleibende Limitierung durch die Koppelproduktion, da das Verhältnis von Ethylen zu Essigsäure in einem weiten Bereich frei einstellbar ist, ohne dass eine spezielle Reaktorauslegung dafür nötig wäre (z.B. angepasste Raum- oder Lineargeschwindigkeit). Vielmehr können weiterhin etablierte technische Reaktorkonzepte Verwendung finden.

Das Verhältnis von Ethylen zu Essigsäure lässt sich in Ausgestaltungen der Erfindung rasch und flexibel an geänderten Bedarf anpassen. Es ist nur ein geringer zusätzlicher Aufwand zur Produktaufreinigung durch gemeinsame Nutzung bestimmter Verfahrensschritte (z.B. Kondensatabscheidung und Zerlegungsteil) erforderlich. Insbesondere sind keine zusätzlichen Verfahren zur Essigsäure-Herstellung oder ein Zukauf erforderlich. Ein weiterer Feed für die Essigsäure-Herstellung ist nicht notwendig. Ferner besteht keine Notwendigkeit zur Verwendung teurer Edelmetallzusätze (insbesondere Pd), um die Essigsäureselektivität zu steigern. Der Katalysator ist ein üblicher Katalysator, insbesondere ein Mischmetalloxidkatalysator.

Je nach Ausführung ist ein geringerer Aufwand in der Ausführung der Ethan-Ethylen-Trennung bzw. des C2-Splitters erforderlich, da keine vollständige Ethylenabtrennung notwendig ist. Insgesamt ergibt sich ein geringerer Aufwand im Vergleich zum Steamcracker und keine nennenswerten Mengen an Nebenprodukten (z.B. keine oder nur sehr geringe Bildung von Methan, nur Spuren von höheren Kohlenwasserstoffen) werden gebildet. Das Gesamtverfahren gemäß Ausgestaltungen der Erfindung ist also hochselektiv in Bezug auf Essigsäure und Ethylen.

**Patentansprüche**

1. Verfahren (100) zur Herstellung von Ethylen und Essigsäure, bei dem Ethan und Sauerstoff in einem Einsatzgemisch (1) unter Erhalt eines Ethylen, Essigsäure und weitere Komponenten enthaltenden Produktgemischs (2) einer oxidativen katalytischen Dehydrierung (110) unter Verwendung eines zumindest Molybdän, Vanadium und Niob sowie optional Tellur als Mischoxid enthaltenden Katalysators unterworfen werden,

**EP 4 594 276 B1**

- wobei das Einsatzgemisch (1) einen Ethylengehalt von 0,25 bis 30 mol.-% aufweist, und zumindest ein Teil des Ethylengehalts des Einsatzgemischs (1) der oxidativen katalytischen Dehydrierung durch Ethylen gebildet wird, das in dem Produktstrom (2) enthalten ist und in die oxidative katalytische Dehydrierung (110) rückgeführt wird,

- wobei das Produktgemisch (2) oder ein Teil hiervon einer Primäraufbereitung (120-170) unterworfen wird, der ein Folgegemisch (5) entnommen wird, das gegenüber dem Produktgemisch (2) an Kohlendioxid und Wasser abgereichert ist,

- wobei das Folgegemisch (5) oder ein Teil hiervon als Tieftemperaturtrenneinsatz einer Tieftemperaturtrennung (180, 190) zugeführt wird, die eine Demethanisierung (180) und insbesondere eine Ethan-Ethylen-Trennung (190) umfasst,

- wobei zumindest ein Teil des Tieftemperaturtrenneinsatzes als ein Demethanisierungseinsatz der Demethanisierung (180) zugeführt wird und in der Demethanisierung (180) eine schwere Fraktion (6) und eine leichte Fraktion (7) als Kopffraktion gebildet werden,

- wobei die schwere Fraktion (6) gegenüber dem Folgegemisch (5) an Kohlenwasserstoffen mit zwei Kohlenstoffatomen, insbesondere an Ethan und Ethylen in Summe, angereichert und an den leichter als Ethylen siedenden Komponenten abgereichert ist,

- wobei die leichte Fraktion (7) gegenüber dem Folgegemisch (5) an den Kohlenwasserstoffen mit zwei Kohlenstoffatomen abgereichert und an den leichter als Ethylen siedenden Komponenten angereichert ist und einen Gehalt von Ethylen von 1 bis 90 Mol- oder Volumenprozent aufweist,

- wobei die leichte Fraktion (7) oder ein Teil hiervon in die oxidative Dehydrierung (110) rückgeführt wird.

2. Verfahren nach Anspruch 1, bei dem in der leichten Fraktion (7) ein Gehalt von Kohlenmonoxid bei bis zu 75%, ein Gehalt von Methan bei bis zu 25%, und ein Gehalt von Sauerstoff unterhalb der Sauerstoffgrenzkonzentration, insbesondere bei weniger als 5% liegt, wobei die Prozentangaben Mol- oder Volumenprozent darstellen.

3. Verfahren (100) nach Anspruch 1 oder Anspruch 2, bei dem ein Anteil von 1 % bis 35% des in dem Produktstrom (2) der oxidativen katalytischen Dehydrierung insgesamt enthaltenen Ethylens in die oxidative katalytische Dehydrierung (110) rückgeführt wird.

4. Verfahren (100) nach Anspruch 3, bei dem das rückgeführte Ethylen mit in dem Produktgemisch (2) der oxidativen katalytischen Dehydrierung enthaltenem Ethan in die oxidative katalytische Dehydrierung (110) rückgeführt wird.

5. Verfahren (100) nach einem der vorstehenden Ansprüche, bei dem die weiteren Komponenten des Produktgemischs (2) der oxidativen katalytischen Dehydrierung in der oxidativen katalytischen Dehydrierung (110) nicht umgesetztes Ethan, leichter als Ethylen siedende Verbindungen, darunter Kohlenmonoxid, sowie Kohlendioxid und Wasser umfassen, und wobei das Folgegemisch (5) zumindest einen Teil des Ethans, des Ethylens und der leichter als Ethylen siedenden Verbindungen aus dem Produktgemisch (2) enthält.

6. Verfahren (100) nach Anspruch 5, bei dem die Primäraufbereitung (120-170) eine Rohgasbehandlung (150) sowie eine Kohlendioxidentfernung (160) und eine Trocknung (170) umfasst, wobei ein Teil eines der Rohgasbehandlung (150) entnommenen Gasgemischs stromauf der Kohlendioxidentfernung (160) und Trocknung (170) aus der Primäraufbereitung (120-170) ausgeführt und in die oxidative katalytische Dehydrierung (110) rückgeführt wird.

7. Verfahren (100) nach Anspruch 1 , wobei zumindest ein Teil des Tieftemperaturtrenneinsatzes als ein Ethan-Ethylen-Trenneinsatz der Ethan-Ethylen-Trennung (190) zugeführt wird, wobei in der Ethan-Ethylen-Trennung (190) eine gegenüber dem Folgegemisch (5) an Ethan angereicherte und an Ethylen und den leichter als Ethylen siedenden Komponenten abgereicherte schwere Fraktion (C2H6) und eine gegenüber dem Folgegemisch (5) an Ethan abgereicherte und an Ethylen und den leichter als Ethylen siedenden Komponenten angereicherte leichte Fraktion (C2H6) gebildet werden, und wobei die leichte Fraktion oder ein Teil hiervon als ein Demethanisierungseinsatz der Demethanisierung (180) zugeführt wird.

8. Verfahren (100) nach Anspruch 1, bei dem ferner eine oder mehrere Fraktionen, die aus der in der Demethanisierung (180) gebildeten Sumpffraktion (6), der in der Ethan-Ethylen- Trennung (190) gebildeten Kopffraktion (C2H4) und der in der Ethan-Ethylen-Trennung (190) gebildeten Sumpffraktion (C2H6) ausgewählt ist oder sind, zumindest zum Teil oder jeweils zumindest zum Teil in die oxidative Dehydrierung (110) rückgeführt wird oder werden.

9. Verfahren (100) nach Anspruch 8, bei dem die eine oder zumindest eine der mehreren Fraktionen, die aus der in der Demethanisierung (180) gebildeten Kopffraktion (7), der in der Demethanisierung (180) gebildeten Sumpffraktion (6), der in der Ethan-Ethylen-Trennung (190) gebildeten Kopffraktion (C2H4) und der in der Ethan-Ethylen-Trennung

14

(190) gebildeten Sumpffraktion (C2H6) ausgewählt ist oder sind, als oder jeweils als Ethan und Ethylen enthaltende Fraktion gebildet wird oder werden.

10. Verfahren nach einem der vorstehenden Ansprüche, bei dem der Ethan-Ethylen-Trennung ein Ethan und Ethylen enthaltender Seitenstrom (9) entnommen wird, der zumindest zum Teil in die oxidative Dehydrierung (110) rückgeführt wird.

11. Verfahren (100) nach einem der vorstehenden Ansprüche, bei dem zumindest ein Teil des in dem Produktgemisch (2) enthaltenen Ethylens in einen offenen Kältemittelkreislauf überführt wird, wobei zumindest ein Teil des Ethylens aus dem offenen Kältekreislauf ausgeführt und zur Bereitstellung zumindest eines Teils des Ethylenanteils des Einsatzgemischs (1) in die oxidative katalytische Dehydrierung (110) rückgeführt wird.

12. Verfahren (100) nach einem der vorstehenden Ansprüche, bei dem zumindest ein zur Bereitstellung zumindest eines Teils des Ethylenanteils des Einsatzgemischs (1) in die oxidative katalytische Dehydrierung (110) rückgeführter Stoffstrom einer Aufbereitung unterworfen wird.

13. Verfahren zur Herstellung einer Zielverbindung unter Verwendung von Ethylen und Essigsäure, **dadurch gekennzeichnet, dass** zur Bereitstellung zumindest eines Teils des Ethylens und der Essigsäure ein Verfahren nach einem der vorstehenden Ansprüche verwendet wird.

**Claims**

1. A method (100) for the production of ethylene and acetic acid, in which ethane and oxygen in a feed mixture (1) are subjected to an oxidative catalytic dehydrogenation (110) using a catalyst containing at least molybdenum, vanadium and niobium and optionally tellurium as a mixed oxide to obtain a product mixture (2) containing ethylene, acetic acid and other components,

   - wherein the feed mixture (1) has an ethylene content of 0.25 to 30 mol%, and at least part of the ethylene content of the feed mixture (1) of the oxidative catalytic dehydrogenation is formed by ethylene contained in the product stream (2) and recycled into the oxidative catalytic dehydrogenation (110),
   - wherein the product mixture (2) or a part thereof is subjected to a primary processing (120-170) from which a subsequent mixture (5) is extracted which is depleted of carbon dioxide and water compared to the product mixture (2),
   - wherein the subsequent mixture (5) or a part thereof is fed as a low-temperature separation feed to a low-temperature separation (180, 190) which includes demethanization (180) and in particular an ethane-ethylene separation (190),
   - wherein at least part of the low-temperature separation feed is fed to the demethanization (180) as a demethanization feed and a heavy fraction (6) and a light fraction (7) are formed as the overhead fraction in the demethanization (180),
   - wherein the heavy fraction (6) is enriched with hydrocarbons having two carbon atoms, in particular with ethane and ethylene overall, compared to the subsequent mixture (5), and is depleted of the components having higher boiling points than ethylene,
   - wherein the light fraction (7) is depleted of hydrocarbons having two carbon atoms and enriched with components having higher boiling points than ethylene compared to the subsequent mixture (5) and has an ethylene content of 1 to 90 molar or volume percent,
   - wherein the light fraction (7) or part thereof is recycled into the oxidative dehydrogenation (110).

2. The method according to claim 1, wherein the light fraction (7) contains up to 75% carbon monoxide, up to 25% methane, and contains oxygen below the limit oxygen concentration, in particular less than 5%, wherein the percentages are molar or volume percent.

3. The method (100) according to claim 1 or claim 2, wherein a proportion of 1% to 35% of the total ethylene contained in the product stream (2) of the oxidative catalytic dehydrogenation is recycled into the oxidative catalytic dehydrogenation (110).

4. The method (100) according to claim 3, wherein the recycled ethylene is recycled together with ethane contained in the product mixture (2) of the oxidative catalytic dehydrogenation into the oxidative catalytic dehydrogenation (110).

**5.** The method (100) according to any of the preceding claims, wherein the further components of the product mixture (2) of the oxidative catalytic dehydrogenation in the oxidative catalytic dehydrogenation (110) comprise unreacted ethane, compounds having lower boiling points than ethylene, including carbon monoxide, as well as carbon dioxide and water, and wherein the subsequent mixture (5) contains at least some of the ethane, the ethylene and the compounds having lower boiling points than ethylene from the product mixture (2).

**6.** The method (100) according to claim 5, wherein the primary processing (120-170) comprises a raw gas treatment (150) as well as a carbon dioxide removal (160) and a drying (170), wherein a portion of a gas mixture taken from the raw gas treatment (150) is extracted from the primary processing (120-170) upstream of the carbon dioxide removal (160) and drying (170) and recycled into the oxidative catalytic dehydrogenation (110).

**7.** The method (100) according to claim 1, wherein at least a part of the low-temperature separation feed is fed to the ethane-ethylene separation (190) as an ethane-ethylene separation feed, wherein in the ethane-ethylene separation (190) a heavy fraction (C2H6) that is enriched with ethane and depleted of ethylene and the components having lower boiling points than ethylene compared to the subsequent mixture (5) and a light fraction (C2H6) depleted of ethane and enriched with ethylene and the components having lower boiling points than ethylene compared to the subsequent mixture (5) are formed, and wherein the light fraction or a part thereof is fed to the demethanization (180) as a demethanization feed.

**8.** The method (100) according to claim 1, wherein one or more fractions selected from the bottoms fraction (6) formed in the demethanization (180), the overhead fraction (C2H4) formed in the ethane-ethylene separation (190) and the bottoms fraction (C2H6) formed in the ethane-ethylene separation (190) are at least partially recycled or are each at least partially recycled into the oxidative dehydrogenation (110).

**9.** The method (100) according to claim 8, wherein one or at least one of the plurality of fractions which is or are selected from the overhead fraction (7) formed in the demethanization (180), the bottoms fraction (6) formed in the demethanization (180), the overhead fraction (C2H4) formed in the ethane-ethylene separation (190) and the bottoms fraction (C2H6) formed in the ethane-ethylene separation (190), is or are formed as a fraction containing ethane and ethylene.

**10.** The method according to any of the preceding claims, wherein a side stream (9) containing ethane and ethylene is drawn from the ethane-ethylene separation and is at least partially recycled into the oxidative dehydrogenation (110).

**11.** The method (100) according to any of the preceding claims, wherein at least a part of the ethylene contained in the product mixture (2) is transferred into an open refrigerant circuit, wherein at least a part of the ethylene is removed from the open refrigerant circuit and is recycled into the oxidative catalytic dehydrogenation (110) to provide at least a part of the ethylene content of the feed mixture (1).

**12.** The method (100) according to any of the preceding claims, wherein at least one material stream that is recycled into the oxidative catalytic dehydrogenation (110) to provide at least a part of the ethylene content of the feed mixture (1) is subjected to processing.

**13.** The method for the preparation of a target compound using ethylene and acetic acid, **characterized in that** a method according to any of the preceding claims is used to provide at least a portion of the ethylene and the acetic acid.

**Revendications**

**1.** Procédé (100) pour la production d'éthylène et d'acide acétique, dans lequel de l'éthane et de l'oxygène sont soumis à une déshydrogénation catalytique oxydante (110) dans un mélange d'alimentation (1) pour obtenir un mélange de produits (2) contenant de l'éthylène, de l'acide acétique et d'autres composants, à l'aide d'un catalyseur contenant au moins du molybdène, du vanadium et du niobium, et éventuellement du tellure comme oxyde mixte,

- dans lequel le mélange d'alimentation (1) présente une teneur en éthylène allant de 0,25 à 30 % en moles, et au moins une partie de la teneur en éthylène du mélange d'alimentation (1) de la déshydrogénation catalytique oxydante est formée par de l'éthylène qui est contenu dans le flux de produits (2) et recyclé dans la déshydrogénation catalytique oxydante (110),
- dans lequel le mélange de produits (2) ou une partie de celui-ci est soumis à un traitement primaire (120-170) duquel est prélevé un mélange subséquent (5) qui est appauvri en dioxyde de carbone et en eau par rapport au mélange de produits (2),
- dans lequel le mélange subséquent (5) ou une partie de celui-ci est amené en tant que charge d'alimentation de séparation à basse température à une séparation à basse température (180, 190) qui comprend une déméthanisation (180) et en particulier une séparation éthane-éthylène (190),
- dans lequel au moins une partie de la charge d'alimentation de séparation à basse température est amenée en tant que charge d'alimentation de déméthanisation à la déméthanisation (180), et dans la déméthanisation (180), une fraction lourde (6) et une fraction légère (7) sont formées en tant que fraction de tête,
- dans lequel, par rapport au mélange subséquent (5), la fraction lourde (6) est enrichie en hydrocarbures comportant deux atomes de carbone, en particulier en éthane et en éthylène au total, et appauvrie en composants à point d'ébullition plus bas que celui de l'éthylène,
- dans lequel, par rapport au mélange subséquent (5), la fraction légère (7) est appauvrie en hydrocarbures comportant deux atomes de carbone et enrichie en composants à point d'ébullition plus bas que celui de l'éthylène et présente une teneur en éthylène allant de 1 à 90 % en moles ou en volume,
- dans lequel la fraction légère (7) ou une partie de celle-ci est recyclée dans la déshydrogénation oxydante (110).

2. Procédé selon la revendication 1, dans lequel, dans la fraction légère (7), une teneur en monoxyde de carbone est inférieure ou égale à 75 %, une teneur en méthane est inférieure ou égale à 25 %, et une teneur en oxygène est inférieure à la concentration limite en oxygène, en particulier inférieure à 5 %, dans lequel les pourcentages sont des pourcentages molaires ou volumiques.

3. Procédé (100) selon la revendication 1 ou la revendication 2, dans lequel une proportion allant de 1 % à 35 % de l'éthylène total contenu dans le flux de produits (2) de la déshydrogénation catalytique oxydante est recyclée dans la déshydrogénation catalytique oxydante (110).

4. Procédé (100) selon la revendication 3, dans lequel l'éthylène recyclé est recyclé dans la déshydrogénation catalytique oxydante (110) avec de l'éthane contenu dans le mélange de produits (2) de la déshydrogénation catalytique oxydante.

5. Procédé (100) selon l'une des revendications précédentes, dans lequel les autres composants du mélange de produits (2) de la déshydrogénation catalytique oxydante comprennent de l'éthane n'ayant pas réagi dans la déshydrogénation catalytique oxydante (110), des composés à point d'ébullition plus bas que celui de l'éthylène, dont le monoxyde de carbone, ainsi que du dioxyde de carbone et de l'eau, et dans lequel le mélange subséquent (5) contient au moins une partie de l'éthane, de l'éthylène et des composés à point d'ébullition plus bas que celui de l'éthylène provenant du mélange de produits (2).

6. Procédé (100) selon la revendication 5, dans lequel le traitement primaire (120-170) comprend un traitement de gaz brut (150) ainsi qu'une élimination de dioxyde de carbone (160) et un séchage (170), dans lequel une partie d'un mélange de gaz prélevé du traitement de gaz brut (150) est prélevée en amont de l'élimination de dioxyde de carbone (160) et du séchage (170) à partir du traitement primaire (120-170) et est recyclée dans la déshydrogénation catalytique oxydante (110).

7. Procédé (100) selon la revendication 1, dans lequel au moins une partie de la charge d'alimentation de séparation à basse température est amenée à la séparation éthane-éthylène (190) en tant que charge d'alimentation de séparation éthane-éthylène, dans lequel, dans la séparation éthane-éthylène (190), une fraction lourde (C2H6) enrichie en éthane et appauvrie en éthylène et en composants à point d'ébullition plus bas que celui de l'éthylène par rapport au mélange subséquent (5) et une fraction légère (C2H6) appauvrie en éthane et enrichie en éthylène et en composants à point d'ébullition plus bas que celui de l'éthylène par rapport au mélange subséquent (5) sont formées, et dans lequel la fraction légère ou une partie de celle-ci est amenée à la déméthanisation (180) en tant que charge d'alimentation de déméthanisation.

8. Procédé (100) selon la revendication 1, dans lequel, en outre, une ou plusieurs fractions choisies parmi la fraction de fond (6) formée dans la déméthanisation (180), la fraction de tête (C2H4) formée dans la séparation éthane-éthylène (190) et la fraction de fond (C2H6) formée dans la séparation éthane-éthylène (190) sont recyclées, au moins en partie ou respectivement au moins en partie, dans la déshydrogénation oxydante (110).

9. Procédé (100) selon la revendication 8, dans lequel la fraction ou au moins l'une des fractions choisies parmi la fraction de tête (7) formée dans la déméthanisation (180), la fraction de fond (6) formée dans la déméthanisation (180), la fraction de tête (C2H4) formée dans la séparation éthane-éthylène (190) et la fraction de fond (C2H6) formée dans la séparation éthane-éthylène (190) est formée en tant que fraction contenant de l'éthane et de l'éthylène, ou respectivement en tant que telle.

10. Procédé selon l'une des revendications précédentes, dans lequel est prélevé de la séparation éthane-éthylène un flux latéral (9) contenant de l'éthane et de l'éthylène, lequel est recyclé au moins en partie dans la déshydrogénation oxydante (110).

11. Procédé (100) selon l'une des revendications précédentes, dans lequel au moins une partie de l'éthylène contenu dans le mélange de produits (2) est transférée dans un circuit ouvert de réfrigérant, dans lequel au moins une partie de l'éthylène est prélevée hors du circuit ouvert de réfrigérant et recyclée dans la déshydrogénation catalytique oxydante (110) pour la fourniture d'au moins une partie de la fraction éthylène du mélange d'alimentation (1).

12. Procédé (100) selon l'une des revendications précédentes, dans lequel au moins un flux de matière recyclé dans la déshydrogénation catalytique oxydante (110) pour la fourniture d'au moins une partie de la fraction éthylène du mélange d'alimentation (1) est soumis à un traitement.

13. Procédé pour la production d'un composé cible à l'aide d'éthylène et d'acide acétique, **caractérisé en ce que,** pour la fourniture d'au moins une partie de l'éthylène et de l'acide acétique, un procédé selon l'une des revendications précédentes est utilisé.

**Fig. 1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2002082445 A1
- WO 2018115416 A1
- EP 3519377 B1
- WO 2019243480 A1
- WO 2018115418 A1
- EP 3558910 B1
- US 10017432 B2
- US 9963412 B2
- WO 2020187572 A1
- US 5907056 A
- US 6258992 A
- EP 1140355 B1
- US 6030920 A

**In der Beschreibung aufgeführte Nicht-Patentliteratur**

- Light Alkanes Oxidation: Targets Reached and Current Challenges. **IVARS, F.** ; **LÖPEZ NIETO, J. M.** Handbook of Advanced Methods and Processes in Oxidation Catalysis: From Laboratory to Industry. Imperial College Press, 2014, 767-834
- **GÄRTNER, C.A. et al.** Oxidative Dehydrogenation of Ethane: Common Principles and Mechanistic Aspects. *ChemCatChem*, 2013, vol. 5 (11), 3196-3217
- **X. LI** ; **E. IGLESIA**. Kinetics and Mechanism of Ethane Oxidation to Acetic Acid on Catalysts Based on Mo-V-Nb Oxides. *J. Phys. Chem. C*, 2008, vol. 112, 15001-15008
- **F. CAVANI et al.** Oxidative dehydrogenation of ethane and propane: How far from commercial implementation?. *Catal. Today*, 2007, vol. 127, 113-131
- **C. LE BERRE** ; **P. SERP** ; **P. KALCK** ; **G. P. TORRENCE**. Acetic Acid. *Ullmann's Encyclopedia of Industrial Chemistry*, 2014